# EUROPEAN PATENT APPLICATION

(11) **EP 2 295 535 A1**
(43) Date of publication of application: **16.03.2011**
(21) Application number: 09170124.3
(22) Date of filing: 11.09.2009
(51) Int. Cl.: C12N 1/20, C12N 1/38, A23L 1/03, A61K 35/74, C12R 1/225

(54) **Probiotic material**

(71) Applicant: Mead Johnson Nutrition Company, Evansville, IN 47721-0001 (US)
(72) Inventor: The designation of the inventor has not yet been filed
(74) Representative: Hatzmann, Martin

(57) **Abstract**

Disclosed is a process for the preparation of a non-viable probiotic composition having anti-allergic (i.e. allergy preventive and/or anti-allergic in a therapeutic sense, and/or anti-inflammatory properties, the process comprising the steps of (a) subjecting LGG to cultivation in a suitable culture medium using a batch process; (b) harvesting the culture supernatant at a late exponential phase of the cultivation step; (c) removing liquid contents from the culture supernatant so as to obtain the composition. Also disclosed is a composition comprising a proteinaceous mixture obtainable by the above process, and the use thereof in a dietetic product preferably for targeting expecting mother's, infants, or children as well as application in food for specific medical purposes.

## Description

### Field of the Invention

The invention pertains to a method of harvesting non-viable, biologically active materials from the probiotic bacterial strain *Lactobacillus rhamnosus* Goldin Gorbach (LGG). Particularly, the invention pertains to a process for the preparation of an anti-allergic and/or anti-inflammatory probiotic material obtainable by said harvesting method, and to dietetic or nutritional products comprising said probiotic material.

### Background of the Invention

Lactobacillus GG (Lactobacillus G.G., strain ATCC 53103) is a bacterium that occurs naturally in the human digestive tract. It is a much studied bacterium, of generally recognized health benefit. It is widely recognized as a probiotic, and consequently incorporated into many nutritional products, such as dairy products, nutritional supplements, infant formula, and the like.

Probiotics are currently defined in the art as live microorganisms which when administered in adequate amounts confer a health benefit on the host. However, the live nature of probiotics brings about challenges when incorporating them into nutritional products. These challenges may differ in order of magnitude depending on, *inter alia,* the type of probiotic strain used, the health status of the individual receiving the product, or both. Also from a process technology point of view, considerable hurdles need to be overcome when incorporating live microorganism in products. This particularly plays a role if one were to incorporate probiotics in longlife products, e.g. powdered products such as infant formula. Also, the challenges increase with the increasing complexity of nutritional product matrices.

Particularly with reference to dietetic products for pregnant women, infants and children, the use of probiotic bacteria is subject to further evaluation of the safety and efficacy. Specific safety questions concern the possible effects on nutrient utilization, the exclusion of transfer of antibiotic resistance, and the short and long term effects on intestinal colonization, immune response, and infections. Whilst this does not mean that probiotics could not be used in such products, it adds to the practical complexity of using live or otherwise viable bacteria.

On the other hand, especially in the event of dietetic products for infants and children, an important demand exists for providing the beneficial effects of probiotics. Moreover, ensuring the stability and vitality of viable bacteria in nutritional products that are made available through retail or hospital channels and exposed to ambient temperatures is particularly challenging. Use of bacterial products, through the application of (processed) culture supernatants in this respect would provide considerable advantages.

The gut microflora in infants is known to be far less developed than that of an adult. While the microflora of the adult human consists of more than 10¹³ microorganisms and nearly 500 species, some being harmful and some being beneficial, the microflora of an infant contains only a fraction of those microorganisms, both in absolute number but also species diversity. Infants are born with a sterile gut, but acquire intestinal flora from the birth canal, their initial environment, and what they ingest. Because the gut microflora population is very unstable in early neonatal life, it is often difficult for the infant's gut to maintain the delicate balance between harmful and beneficial bacteria, thus reducing the ability of the immune system to function normally.

It is especially difficult for formula-fed infants to maintain this balance due to the differences between the bacterial species in the gut of a formula-fed and breast-fed infant. The stool of breast-fed infants contains predominantly Bifidobacterium, with Streptococcus and Lactobacillus as less common contributors. In contrast, the microflora of formula-fed infants is more diverse, containing Bifidobacterium and Bacteroides as well as the more pathogenic species, Staphylococcus, Escherichia coli, and Clostridia. The varied species of Bifidobacterium in the stools of breast-fed and formula-fed infants differ as well. A variety of factors have been proposed as the cause for the different fecal flora of breast-fed and formula-fed infants, including the lower content and different composition of proteins in human milk, a lower phosphorus content in human milk, the large variety of oligosaccharides in human milk, and numerous humoral and cellular mediators of immunologic function in breast milk. Agostoni, et al., Probiotic Bacteria in Dietetic Products for Infants: A Commentary by the ESPGHAN Committee on Nutrition, J. Pediatr. Gastro. Nutr. 38:365-374 (April 2004).

The establishment of a normal intestinal bacterial flora has important implications for health and disease. The major function of the gut n1ierohiota, from the host's point of view, is to prevent colonization of the intestine with pathogenic organisms and to inhibit the proliferation of potentially pathogenic microorganisms, increasing the natural resistance to infectious diseases of the intestinal tract. Probiotics exert this effect by preventing the binding of pathogenic bacteria to the enterocyte, either directly by producing antimicrobial compounds or indirectly by altering the pH of the intestinal lumen through the synthesis of short-chain volatile fatty acids.

In addition, the normal flora might stimulate gastrointestinal immunity, enhance mucosal IgA production, stimulate the local production of anti-inflammatory cytokines and reduce the generation of proinflammatory cytokines characteristic of allergic inflammation. A survey of the most relevant studies concerning the use of probiotics in food allergy, atopic dermatitis, and primary prevention of atopy has shown that probiotic therapy alleviates allergic inflammation as demonstrated by the control of clinical symptoms and the reduction of local and systemic inflammatory markers (Miraglia del Giudice M, De Luca MG. The role of probiotics in the clinical management of food allergy and atopic dermatitis. J. Clin Gastroenterol 2004;38(6 Suppl):S84-5; Prescott S , Bjorksten B, Probiotics for the prevention or treatment of allergic disease. J. Allergy Clin Immunol. 2007;120:255-262).

In view of the foregoing, it will be understood that it is generally desired to provide nutritional products, dietetic products and, particularly infant formula, with probiotics. The term "infant formula" refers to a composition that satisfies the nutrient requirements of an infant by being a substitute for human milk.

It will also be understood that it is desired, particularly in infant formula, to incorporate probiotics without necessarily incorporating live or otherwise viable bacteria. In fact, this calls for "non-viable probiotics."

Research on such non-viable probiotics is ongoing. However, as stated e.g. in a paper by Tao et al. (Am J. Physiol Cell Physiol. 290: C1018-C1030 (2006), although probiotics appear to improve the course of many illnesses, their mechanisms of action are poorly understood. Attempts have been made only recently to understand the mechanisms behind their actions and interactions with the host cell. Many different possible mechanisms have been proposed, including upregulation of mucus production, improvement in epithelial barrier function, increase in IgA production, and increased competition for adhesion sites on intestinal epithelia, as well as the production of organic acids, ammonia, hydrogen peroxide, and bacteriocins, which inhibit the growth of pathogenic bacteria. In this regard Tao et al. present a study on certain effects of soluble factors from LGG. Certain bioactivity was concluded to reside in a low-molecular weight fraction (MW < 10 kDa) of an LGG-CM ultrafiltrate. Of similar teaching is US 2007/123460. Herein bacteria-free, probioticderived compounds, and particularly a low-molecular weight fraction (MW < 10 kDa) instead of live bacteria are used in the treatment of particularly inflammatory bowel diseases (IBDs). Other reference touching on non-viable probiotics are e.g. US 2004/208863, which generally concerns anti-inflammatory action of bacteria, and secreted products therefrom, and US 7052896 which relates to anti-inflammatory and anti-allergic properties of peptides produced by Lactobacillus rhamnosus species.

US 6,506,389 describes a protein obtainable from a non pathogenic microorganism, said protein having mucosa binding activity and a molecular weight of 20-40 kD, preferably 20-30 kD or an equivalent polypeptide thereof. The gist of the disclosure is to provide a screening method for identifying proteins and polypeptides capable of specifically binding mucosa.

None of the prior art references presents a suitably straightforward method of harvesting from LGG a non-viable probiotic material that supports anti-allergic and/or anti-inflammatory activity.

Further, the harvesting of secreted bacterial products brings about a problem that the culture media cannot easily be deprived of undesired components. This specifically relates to nutritional products for relatively vulnerable subjects, such as infant formula or clinical nutrition. This problem is not incurred if specific components from a culture supernatant are first isolated, purified, and then applied in a nutritional product. However, it is desired to make use of a more complete cultural supernatant. This would serve to provide , a composition better reflecting the natural action of the probiotic (i.e. LGG). Currently, however, one cannot just use the culture supernatant itself as a basis for non-viable probiotic materials to be specifically used in infant formula and the like. It is thus moreover desired to provide a method to resolve this.

### Summary of the Invention

In order to better address one or more of the foregoing desires, the invention, in one aspect, presents a process for the preparation of a non-viable probiotic composition having anti-allergic and/or anti-inflammatory properties, the process comprising the steps of (a) subjecting LGG to cultivation in a suitable culture medium using a batch process; (b) harvesting the culture supernatant at a late exponential phase of the cultivation step, which phase is defined with reference to the second half of the time between the lag phase and the stationary phase of the LGG batch-cultivation process; (c) optionally removing low molecular weight constituents from the supernatant so as to retain molecular weight constituents above 6 kDa; (d) removing liquid contents from the spent culture supernatant so as to obtain the composition.

In another aspect, the invention resides in a method of harvesting from an LGG culture medium a composition having anti-allergic and/or anti-inflammatory activity, the method comprising growing LGG in a suitable culture medium, determining the late-exponential phase of LGG population growth, and separating the culture supernatant in said late-exponential phase from the bacterial culture.

In still another aspect, the invention provides a composition comprising a non-viable probiotic material obtainable from a culture supernatant in a late-exponential phase of an LGG batch-cultivation process.

In further aspects, the invention provides a dietetic product comprising a non-viable probiotic composition obtainable from a culture supernatant in a late-exponential phase of an LGG batch-cultivation process, as well as the use of the foregoing composition as an additive in a nutritional product.

In a yet another aspect, the invention resides in the use of the foregoing non-viable probiotic composition in providing a nutritional product that possesses anti-allergic and/or anti-inflammatory properties.

In a still further aspect, the invention provides a method of treatment or prevention of an allergic and/or inflammatory disease in a subject, the method comprising the administration to said subject of an effective amount of a composition comprising a non-viable probiotic material obtainable from a culture supernatant in a late-exponential phase of an LGG batch-cultivation process.

Also, in another aspect, the invention presents depletion of the LGG culture medium of Tween 80 and/or instead using an oily ingredient selected from the group consisting of oleic acid, linseed oil, olive oil, rape seed oil, sunflower oil and mixtures thereof, in a bacterial culture medium, as a functional replacement for non-ionic emulsifiers such as polysorbates.

### Brief description of the drawings

Fig. 1 shows a graph representing the increase of the LGG population, with time, upon cultivation; Herein Fig. 1a depicts this with reference to optical density (OD600) as well as pH change of the culture media and Fig. 1b presents the bacterial counts determined by plating techniques.
Fig. 2 shows the production of cytokine IL-10 for the different phases MJ1, MJ2, MJ3) of LGG culture harvests;
Fig.3 depicts the timeline of the *in vivo* ovalbumin (OVA) sensitation; model used in testing the composition of the invention;
Fig. 4 depicts microscope images of stained lung tissue in mice subjected to the OVA model and oral treatment with different compositions including viable LGG and LGG supernatant;
Fig. 5 shows a diagram representing the allergic cells infiltration in the lungs of OVA allergic animals;
Fig. 6 (a-c) displays diagrams representing the *in vitro* responses for three different cytokines relevant to the inflammatory process in allergic disease;
Fig. 7 presents a scheme for an *in vivo* investigation on the perinatal administration of LGG culture supernatant of the invention;
Fig. 8 displays the results on allergic reactivity upon perinatal administration of LGG culture supernatant of the invention;

### Detailed description of embodiments

The invention, in a broad sense, is based on the insight that from LGG batch cultivation a culture supernatant (which can also be referred to as "spent medium") can be harvested that possesses anti-allergic activity. In the context of this invention, the term "anti-allergic" includes "allergy preventive activity as well as anti-allergic therapeutic activity."

Without wishing to be bound by theory, the present inventors believe that this activity can be attributed to the mixture of components (including proteinaceous materials, and possibly including (exo)polysaccharide materials) as found released into the culture medium at a late stage of the exponential (or "log") phase of batch cultivation of LGG. The composition will be hereinafter referred to as "culture supernatant of the invention."

LGG is a probiotic strain isolated from healthy human intestinal flora. It was disclosed in U.S. Patent No. 5,032,399 to Gorbach, et al., which is herein incorporated in its entirety, by reference thereto. LGG is resistant to most antibiotics, stable in the presence of acid and bile, and attaches avidly to mucosal cells of the human intestinal tract. It survives for 1-3 days in most individuals and up to 7 days in 30% of subjects. In addition to its colonization ability, LGG also beneficially affects mucosal immune responses. LGG is deposited with the depository authority American Type Culture Collection under accession number ATCC 53103.

The stages recognized in batch cultivation of bacteria are known to the skilled person. These are the "lag," the "log" ("logarithmic" or "exponential"), the "stationary" and the "death" (or "logarithmic decline") phases. In all phases during which live bacteria are present, the bacteria metabolize nutrients from the media, and secrete (exert, release) materials into the culture medium. The composition of the secreted material at a given point in time of the growth stages is not generally predictable.

In the present invention, secreted materials are harvested from a late exponential phase. The late exponential phase occurs in time after the mid exponential phase (which is halftime of the duration of the exponential phase, hence the reference to the late exponential phase as being the second half of the time between the lag phase and the stationary phase). In particular, the term "late exponential phase" is used herein with reference to the latter quarter portion of the time between the lag phase and the stationary phase of the LGG batch-cultivation process. Preferably, in accordance with the invention, harvesting of the culture supernatant is at a point in time of 75% to 85% of the duration of the exponential phase, and most preferably is at about ⁵/₆ of the time elapsed in the exponential phase.

The term "cultivation" or "culturing" refers to the propagation of micro-organisms, in this case LGG, on or in a suitable medium. Such a culture medium can be of a variety of kinds, and is particularly a liquid broth, as customary in the art. A preferred broth, e.g., is MRS broth as generally used for the cultivation of lactobacilli. MRS broth generally comprises polysorbate, acetate, magnesium and manganese, which are known to act as special growth factors for lactobacilli, as well as a rich nutrient base. A typical composition comprises (amounts in g/liter): peptone from casein 10.0; meat extract 8.0; yeast extract 4.0; D(+)-glucose 20.0; dipotassium hydrogen phosphate 2.0; Tween^{®} 80 1.0; triammonium citrate 2.0; sodium acetate 5.0; magnesium sulfate 0.2; manganese sulfate 0.04.

A preferred use of the culture supernatant of the invention is in infant formula. In order for the invention to be of full use herein, it is desired to ensure that the composition harvested from LGG cultivation does not contain components (as may present in the culture medium) that are not desired, or legally allowed, in such formula. With reference to polysorbate regularly present in MRS broth, media for the culturing of bacteria may include an emulsifying non-ionic surfactant, e.g. on the basis of polyethoxylated sorbitan and oleic acid (typically available as Tween® polysorbates, such as Tween® 80). Whilst these surfactants are frequently found in food products, e.g. ice cream, and are generally recognized as safe, they are not in all jurisdictions considered desirable, or even acceptable for use in nutritional products for relatively vulnerable subjects, such as infant formula or clinical nutrition.

The present invention thus, in a preferred embodiment, also pertains to using culture media in which the aforementioned polysorbates can be avoided. To this end, a preferred culture medium of the invention is devoid of Tween 80 and can comprise an oily ingredient selected from the group consisting of oleic acid, linseed oil, olive oil, rape seed oil, sunflower oil and mixtures thereof. It will be understood that the full benefit of the oily ingredient is attained if the presence of a polysorbate surfactant is essentially or entirely avoided.

Most preferably, an MRS medium is devoid of Tween 80 and comprises, in addition to one or more of the foregoing oils, peptone (typically 10 g/L), meat extract (typically 8 g/L), yeast extract (typically 4 g/L), D(+) glucose (typically 20 g/L), dipotassium hydrogen phosphate (typically 2 g/L), sodium acetate trihydrate (typically 5 g/L), triammonium citrate (typically 2 g/L), magnesium sulfate heptahydrate (typically 0.2 g/L) and manganous sulfate tetrahydrate (typically 0.05 g/L).

The culturing is generally performed at a temperature of 20 °C to 45°C, preferably at 35 °C to 40°C, and most preferably at 37°C.

The preferred time point during cultivation for harvesting the culture supernatant, i.e., in the aforementioned late exponential phase, can be determined, e.g. based on the OD600nm and glucose concentration. OD600 refers to the optical density at 600 nm, which is a known density measurement that directly correlates with the bacterial concentration in the culture medium.

In addition to the foregoing, it should be noted that the batch cultivation of lactobacilli, including LGG, is common general knowledge available to the person skilled in the art. These methods thus do not require further elucidation here.

Preferably, the composition of the invention is produced by large scale fermentation (e.g. in a more than 100 L fermentor, preferably about 200 L or higher).

The composition of the invention can be harvested by any known technique for the separation of culture supernatant from a bacterial culture. Such techniques are well-known in the art and include, e.g., centrifugation, filtration, sedimentation, and the like.

The supernatant may be used immediately, or be stored for future use. In the latter case, the supernatant will generally be refrigerated, frozen or lyophilized. The supernatant may be concentrated or diluted, as desired.

The composition as harvested in accordance with the invention, is believed to be a proteinaceous composition. The term "proteinaceous" is known to the skilled person, and indicates that the composition comprises one or more of peptides, proteins, or other compounds comprising amino acid residues.

As to the chemical substances, the composition of the culture supernatant of the invention is believed to be a mixture of a plurality of amino acids, oligo- and polypeptides, and proteins, of various molecular weights.

It is emphasized, as different from the art, that the invention preferably pertains to the entire, i.e. unfractionated culture supernatant. The judicious choice of harvesting at the above-mentioned late exponential phase, and the retention of virtually all components of the supernatant, are believed to contribute to the surprising results obtained therewith, particularly in view of anti-allergic activity and more particularly in view of such activity in infants and neonates, and upon perinatal administration to pregnant respectively lactating women.

The entire culture supernatant is more specifically defined as substantially excluding low molecular weight components, generally below 6 kDa. This relates to the fact that the composition preferably does not include lactic acid and/or lactate salts. The preferred supernatant of the invention thus has a molecular weight of greater than 6kDa, as this is the typical supernatant obtained upon the removal of lactic acid and lactate salts. This usually involves filtration or column chromatography. As a matter of fact, the retentate of this filtration represents a molecular weight range of greater than 6 kDa (in other words, constituents of below 6 kDa are filtered off).

The composition of the supernatant of the invention will generally not only be proteinaceous, but also comprises polysaccharides, particularly exopolysaccharides (high molecular-weight polymers composed of sugar residues as produced by LGG). Without wishing to be bound by theory, the present inventors believe that the ratio between the amounts of proteinaceous materials and the amounts of carbohydrate materials as harvested from the late exponential phase as discussed above, contributes to the anti-allergic nature as compared to compositions as harvested from other stages, e.g. the mid-exponential phase or the stationary phase.

The culture supernatant harvested in accordance with the invention, can be put to use in various ways, so as to benefit from the anti-allergic activity found. Such use will generally involve some form of administration of the composition of the invention to a subject in need thereof. In this respect, the culture supernatant can be used as such, e.g. incorporated into capsules for oral administration, or in a liquid nutritional composition such as a drink, or it can be processed before further use. The latter is preferred.

Such processing generally involves separating the proteinaceous composition from the generally liquid continuous phase of the supernatant. This preferably is done by a drying method, such as spray-drying or freezedrying (lyophilization). Spray-drying is preferred. In a preferred embodiment of the spray-drying method, a carrier material will be added before spray-drying, e.g., maltodextrin DE29. This is believed to be advantageous in view of the production of a dry powder also under conditions in which lactic acid (which is produced by LGG and which is present in the spent culture medium) is a liquid.

The composition of the invention has been found to possess anti-allergic (preventive and/or therapeutic) activity. Anti-allergic activity can be determined, e.g., in a newly developed neonatal mouse model of allergic sensitisation and lung inflammation. This model in fact is an adaptation of the so called OVA model which is widely used to study the immune pathology of allergic diseases and asthma as well as to identify compounds with anti-allergic or anti-inflammatory activity. In addition to using it for its anti-allergic activity, in other embodiments the composition of the invention will be used in the prevention or treatment of a wide array of inflammatory diseases. These allergic and/or inflammatory diseases include, but are not limited to asthma (which is an inflammatory condition that may be allergy-based or not), atopic eczema (which also may be allergy-based or not), food allergy, and allergic rhinitis/conjunctivitis.

In order for the composition of the invention to exert its beneficial, anti-allergic and/or anti-inflammatory effect, it is to be digested by a subject, preferably a human subject. Particularly, in a preferred embodiment, the subject is a pregnant woman, a lactating woman, a neonate, an infant, or a child. As referred to above, the advantages of using a material that could be regarded a "non-viable probiotic," will be benefited from most in dietetic products for infants. The term "infant" means a postnatal human of less than about 1 year old.

It will be understood that digestion by a subject will require the oral administration of the composition of the invention. The form of administration of the composition in accordance with the invention is not critical. In some embodiments, the composition is administered to a subject via tablets, pills, encapsulations, caplets, gel caps, capsules, oil drops, or sachets. In another embodiment, the composition is encapsulated in a sugar, fat, or polysaccharide.

In yet another embodiment, the composition is added to a food or drink product and consumed. The food or drink product may be a children's nutritional product such as a follow-on formula, growing up milk, beverage, milk, yoghurt, fruit juice, fruit-based drink, chewable tablet, cookie, cracker, or a milk powder. In other embodiments, the product may be an infant's nutritional product, such as an infant formula or a human milk fortifier.

The composition of the invention, whether added in a separate dosage form or via a nutritional product, will generally be administered in an amount effective in the treatment or prevention of allergies and/or inflammatory disorders. The effective amount is preferably equivalent to 1x10⁴ to about 1x10¹² cell equivalents of live probiotic bacteria per kg body weight per day, and more preferably 10⁸-10⁹. The back-calculation to cell equivalents is well within the ambit of the skilled person's knowledge.

If the composition of the invention is administered via an infant formula, the infant formula may be nutritionally complete and contain suitable types and amounts of lipid, carbohydrate, protein, vitamins and minerals. The amount of lipid or fat typically may vary from about 3 to about 7 g/100 kcal. Lipid sources may be any known or used in the art, e.g., vegetable oils such as palm oil, soybean oil, palmolein, coconut oil, medium chain triglyceride oil, high oleic sunflower oil, high oleic safflower oil, and the like. The amount of protein typically may vary from about 1 to about 5 g/100 kcal. Protein sources may be any known or used in the art, e.g., non-fat milk, whey protein, casein, soy protein, (partially or extensively) hydrolyzed protein, amino acids, and the like. The amount of carbohydrate typically may vary from about 8 to about 12 g/100 kcal. Carbohydrate sources may be any known or used in the art, e.g., lactose, glucose, corn syrup solids, maltodextrins, sucrose, starch, rice syrup solids, and the like.

Conveniently, commercially available prenatal, premature, infant and children's nutritional products may be used. For example, Expecta® Enfamil®, Enfamil® Premature Formula, Lactofree ®, Nutramigen®, Gentlease®, Pregestimil®, ProSobee®, Enfakid®, Enfaschool®, Enfagrow®, Kindercal® (available from Mead Johnson & Company, Evansville, Ind., U.S.A.) may be supplemented with suitable levels of composition of the inventions and used in practice of the method of the invention.

In one embodiment, the composition of the invention may be combined with one or more viable probiotics. Any viable probiotic known in the art may be acceptable in this embodiment provided it achieves the intended result.

If a viable probiotic is administered in combination with the composition of the invention, the amount of viable probiotic may correspond to between about 1*10⁴ and 1*10¹² colony forming units (cfu) per kg body weight per day. In another embodiment, the viable probiotics may comprise between about 1*10⁶ and 1*10¹² cfu per kg body weight per day. In yet another embodiment, the viable probiotics may comprise about 1*10⁹ cfu per kg body weight per day. In a still further embodiment, the viable probiotics may comprise about 1*10¹⁰ cfu per kg body weight per day.

In another embodiment, the composition of the invention may be combined with one or more prebiotics. A "prebiotic" means a non-digestible food ingredient that stimulates the growth and/or activity of probiotics. Any prebiotic known in the art will be acceptable in this embodiment provided it achieves the desired result. Prebiotics useful in the present invention may include lactulose, gluco-oligosaccharide, inulin, polydextrose, galacto-oligosaccharide, fructo-oligosaccharide, isomaltooligosaccharide, soybean oligosaccharides, lactosucrose, xylooligosacchairde, and gentio-oligosaccharides.

In yet another embodiment of the present invention, the infant formula may contain other active agents such as LCPUFAs. Suitable LCPUFAs include, but are not limited to, [alpha]-linoleic acid, [gamma]-linoleic acid, linoleic acid, linolenic acid, eicosapentanoic acid (EPA), arachidonic acid (ARA) and/or docosohexaenoic acid (DHA). In an embodiment, the composition of the invention is administered in combination with DHA. In another embodiment, the composition of the invention is administered in combination with ARA. In yet another embodiment, the composition of the invention is administered in combination with both DHA and ARA. Commercially available infant formula that contains DHA, ARA, or a combination thereof may be supplemented with the composition of the invention and used in the present invention. For example, Enfamil® LIPIL®, which contains effective levels of DHA and ARA, is commercially available and may be supplemented with the composition of the invention and utilized in the present invention. If included, the effective amount of ARA in an embodiment of the present invention is typically from about 5 mg per kg of body weight per day to about 150 mg per kg of body weight per day. In one embodiment of this invention, the amount varies from about 10 mg per kg of body weight per day to about 120 mg per kg of body weight per day. In another embodiment, the amount varies from about 15 mg per kg of body weight per day to about 90 mg per kg of body weight per day. In yet another embodiment, the amount varies from about 20 mg per kg of body weight per day to about 60 mg per kg of body weight per day. If an infant formula is utilized, the amount of DHA in the infant formula may vary from about 5 mg/100 kcal to about 80 mg/100 kcal. In one embodiment of the present invention, DHA varies from about 10 mg/100 kcal to about 50 mg/100 kcal; and in another embodiment, from about 15 mg/100 kcal to about 20 mg/100 kcal. In a particular embodiment of the present invention, the amount of DHA is about 17 mg/100 kcal. If an infant formula is utilized, the amount of ARA in the infant formula may vary from about 10 mg/100 kcal to about 100 mg/100 kcal. In one embodiment of the present invention, the amount of ARA varies from about 15 mg/100 kcal to about 70 mg/100 kcal. In another embodiment, the amount of ARA varies from about 20 mg/100 kcal to about 40 mg/100 kcal. In a particular embodiment of the present invention, the amount of ARA is about 34 mg/100 kcal. If an infant formula is used, the infant formula may be supplemented with oils containing DHA and ARA using standard techniques known in the art. For example, DHA and ARA may be added to the formula by replacing an equivalent amount of an oil, such as high oleic sunflower oil, normally present in the formula. As another example, the oils containing DHA and ARA may be added to the formula by replacing an equivalent amount of the rest of the overall fat blend normally present in the formula without DHA and ARA. If utilized, the source of DHA and ARA may be any source known in the art such as marine oil, fish oil, single cell oil, egg yolk lipid, brain lipid, and the like. In some embodiments, the DHA and ARA are sourced from the single cell Martek oil, DHASCO®, or variations thereof. The DHA and ARA can be in natural form, provided that the remainder of the LCPUFA source does not result in any substantial deleterious effect on the infant. Alternatively, the DHA and ARA can be used in refined form. In an embodiment of the present invention, sources of DHA and ARA are single cell oils as taught in U.S. Pat. Nos. 5,374,567; 5,550,156; and 5,397,591, the disclosures of which are incorporated herein in their entirety by reference. However, the present invention is not limited to only such oils. In one embodiment, a LCPUFA source which contains EPA is used in combination with at least one composition of the invention. In another embodiment, a LCPUFA source which is substantially free of EPA is used in combination with at least one composition of the invention. For example, in one embodiment of the present invention, an infant formula containing less than about 16 mg EPA/100 kcal is supplemented with the composition of the invention. In another embodiment, an infant formula containing less than about 10 mg EPA/100 kcal is supplemented with the composition of the invention. In yet another embodiment, an infant formula containing less than about 5 mg EPA/100 kcal is supplemented with the composition of the invention.

Another embodiment of the invention includes an infant formula supplemented with the composition of the invention that is free of even trace amounts of EPA. It is believed that the provision of a combination of the composition of the invention with DHA and/or ARA provides complimentary or synergistic effects with regards to the anti-allergic and/or anti-inflammatory properties of formulations containing these agents.

In a further preferred embodiment, the dietetic product of the invention comprises one or more bio-active materials normally present in human breast milk, such as proteins or polysaccharides.

The composition of the invention is preferably used in order to prevent, reduce, ameliorate or treat allergies and/or symptoms thereof.

Allergy is defined as an "abnormal hypersensitivity to a substance which is normally tolerated and generally considered harmless." The symptoms of allergies can range from a runny nose to anaphylactic shock. Nearly 50 million Americans suffer from allergic disease, and the incidence of these illnesses is increasing.

There are two basic phases involved with the allergic response. The first stage involves the development of the early phase of an immediatetype hypersensitivity response to allergens. The first time an allergen meets the immune system, no allergic reaction occurs. Instead, the immune system prepares itself for future encounters with the allergen. Macrophages, which are scavenger cells, and so-called dendritic cells surround and break up the invading allergen. The cells then display the allergen fragments on their cell walls to T lymphocytes, which are the main orchestrators of the body's immune reaction. This cognitive signal plus several non-cognitive signals (e.g. cytokines) activate the naive T-cells and instruct the T-cell differentiation into T-cell effector subpopulations. The key players in the allergic cascade are T-cells of the Th-2 phenotype (TH-2). TH-2 type T-cells are characterized by the secretion of several cytokines including interleukin-4 (IL-4), IL-5 and IL-13. The cytokines IL-4 and IL-13 then activate B lymphocytes to produce antibodies of the subclass E (IgE) that are directed against the particular allergen. The interaction of specific IgE antibodies on the surface of effector cells (mast cells and basophils) with an allergen triggers the early phase of immediate type hypersensitivity responses.

This mast cell activation usually occurs within minutes after the second or additional exposure to an allergen. IgE antibodies on mast cells, constructed during the sensitization phase, recognize the allergen and bind to the invader. Once the allergen is bound to the receptor, granules in the mast cells release their contents. These contents, or mediators, are proinflammatory substances such as histamine, platelet-activating factor, prostaglandins, cytokines and leukotrienes. These mediators actually trigger the allergy attack. Histamine stimulates mucus production and causes redness, swelling, and inflammation. Prostaglandins constrict airways and enlarge blood vessels.

The second phase of the allergic immune response is characterized by infiltration of inflammatory cells, such as eosinophils, into the airways after an allergen exposure. An important linkage between sensitization and inflammation is represented by T-cells that secrete mediators not only involved in IgE synthesis, but also responsible for eosinophil recruitment, activation and survival. The tissue mast cells and neighbouring cells produce chemical messengers that signal circulating basophils, eosinophils, and other cells to migrate into that tissue and help fight the foreign material. Eosinophils secrete chemicals of their own that sustain inflammation, cause tissue damage, and recruit yet more immune cells. This phase can occur anywhere between several hours and several days after the allergen exposure and can last for hours and even days.

Respiratory allergy is a particular type of allergy that affects the respiratory tract. The lining of the airway from the nose to the lungs is similar in structure and is often similarly affected by the allergic process. Therefore, an allergen that affects the nose or sinus also could affect the lungs.

For example, allergic rhinitis, also known as hay fever, is caused by allergic reactions of the mucous membranes in the nose and airway to allergens in the air. Symptoms of allergic rhinitis often include itchy nose, throat and eyes and excessive sneezing. Stuffy or runny nose often follow.

As allergens in one area of the respiratory tract can affect other areas of the respiratory tract, rhinitis in the nasal passages can lead to asthma, which is a much more serious illness that occurs in the lower airways of the lungs. Asthma is characterized by development of airway hyper reactivity, breathlessness, wheezing on exhale, dry cough and a feeling of tightness in the chest. Repeated allergen exposure can sustain the inflammatory immune response in the airways, resulting in a remodelling of the airways, commonly known as chronic asthma. Not everyone with allergic rhinitis will develop asthma symptoms, but a significant number, especially those with recurring, untreated allergies, will show lung inflammation changes. About forty percent of people with allergic rhinitis will actually develop full-blown asthma.

If the nasal inflammation that accompanies allergic rhinitis reaches the sinuses, the result can be an uncomfortable infection called sinusitis, or rhino-sinusitis, in which the sinuses cannot empty themselves of bacteria. Symptoms include nasal congestion, runny nose, sore throat, fever, headache, fatigue and cough, as well as pain in the forehead, behind the cheeks, and even aching teeth and jaw.

Respiratory allergies are one of the most common afflictions of childhood. As with adults, respiratory allergies in children are most likely to appear in the form of allergic rhinitis and asthma.

The prevention of respiratory allergies is especially important in infants and young children, as it appears that early allergic sensitization to allergens is associated with a delay in the maturation of normal immune responses. Additionally, allergic sensitization is generally considered the first step in developing atopic disease. Baena-Cagnani, Role of Food Allergy in Asthma in Childhood, Allergy. Clin. Immun. 1(2):145-149 (2001). Frequently, asthma that begins early in life is associated with atopy, thus early allergic sensitization seems to play an important role in persistent asthma as well. Martinez, F., Development of Wheezing Disorders and Asthma in Preschool Children, Pediatr. 109:362-367 (2002).

Not only is there a strong association between allergic sensitization and asthma, but the association appears to be age- dependent. Although few children become allergen sensitized during the first few years of life, the great majority of those who do become sensitized during this period develop asthma-like symptoms later in life. Martinez, F., Viruses and Atopic Sensitization in the First Years of Life, Am. J. Respir. Crit. Care Med., 162:S95-S99 (2000). Thus, it is important to find ways to prevent early allergen sensitization and prevent respiratory allergies later in life.

There is increasing evidence that many aspects of health and disease are determined not only during infancy, but also during pregnancy. This is especially true with allergic disease, where immune responses at birth implicate intrauterine exposure as a primary sensitization event. For example, allergen-specific T-cells are already present at birth and early sensitization to food allergens are identified as predictors for later development of respiratory allergies. IHi, et al., The Natural Course of Atopic Dermatitis from Birth to Age 7 Years and the Association with Asthma, Clin. Exp. Allergy 27:28-35 (1997). In addition, lung development begins very early after fertilization and continues for at least two or three years after birth. Thus, both prenatal and postnatal airway development are important in the pathogenesis of respiratory allergy in infants and children.

It has also been shown that the human fetus develops IgE-producing B cells early in gestation and is capable of producing IgE antibodies in response to appropriate antigenic stimuli in a manner analogous to the well-recognized IgM responses that are observed in various prenatal infections. Weil, G., et al., Prenatal Allergic Sensitization to Helminth Antigens in Offspring of Parasite-Infected Mothers, J. Clin. Invest. 71 :1124-1129 (1983). This also illustrates the importance of preventing both prenatal and postnatal allergic sensitization to respiratory allergens.

Traditional medications for respiratory allergies include antihistamines, topical nasal steroids, decongestants, and cromolyn solution. As an alternative to traditional medications, probiotics have emerged as possible treatments for certain types of allergies. With reference to the above-mentioned drawbacks of using live or viable probiotics, the present invention is of particular benefit in substituting such probiotics in products that serve to prevent, reduce, ameliorate or treat allergic diseases and/or symptoms thereof. To this end the composition is preferably administered via a dietetic or nutritional product, more preferably a prenatal, infant or children's formula or nutritional composition, a medical food, or a food for specific medical purposes (i.e. a food labelled for a defined medical purpose), most preferably an infant formula, or perinatal nutrition for pregnant or lactating women, as substantially discussed hereinbefore.

The invention will be illustrated hereinafter with reference to the following, non-limiting examples and the accompanying figures.

### Example 1

In a batch fermentation process, LGG was grown under physiological conditions. The pH was kept constant at pH6 by the addition of 33% NaOH, temperature was kept at 37°C. The stirrer speed was 50 rpm, headspace was flushed with N₂. The following culture medium (an adapted MRS Broth) was provided (Table 1).

**Table 1**

| Component | | (g) |
|---|---|---|
| ***Solution 1 (autoclaved separately at 110°C)*** | | |
| | Glucose-H₂O | 66 |
| | Demineralized water | 84 |

| ***Solution 2 (autoclaved at 121 °C)*** | | |
|---|---|---|
| | Tween-80 | 2.0 |
| | Na-acetate·3 H₂O | 10.0 |
| | NH₄Cl | 2.6 |
| | Na₃-Citrate-2 H₂O | 4.8 |
| | K₂HPO₄ | 4.0 |
| | MgSO₄·7 H₂O | 0.4 |
| | MnSO₄·H₂O | 0.08 |
| | Yeast extract (Gistex LS, Powder) | 46 |
| | Demineralized water | 780 |
| ***Total*** | | 1 kg medium |

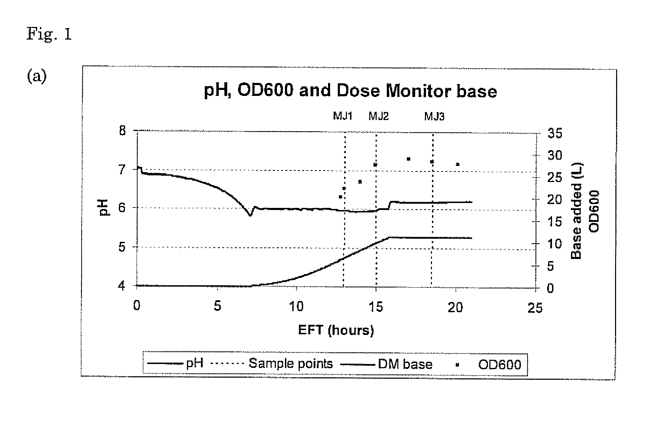

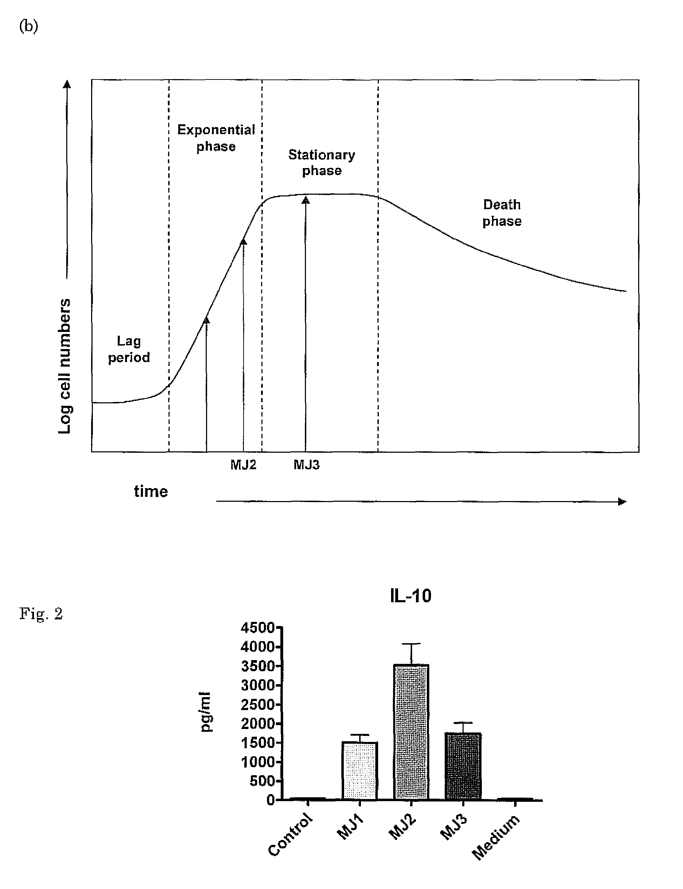

The bacterial growth is depicted in Figures 1 (a) and (b).

Fig. 1a shows the evolution of the pH, the amount of NaOH (33%) titrated (DM base = Dose Monitor base) and OD600 during the LGG fermentation. Reference is made to the legend given in the figure. The pH and OD600 measurements allow a determination of the bacterial growth in the fermenter; herein the addition of NaOH needed to keep pH at 6 correlates with lactate production (i.e. a measure for bacterial metabolic activity) and OD 600 is a density measurement that correlates with the number of bacteria in the fermenter.

In Fig. 1b the vertical axis indicates, on a logarithmic scale, the bacterial count in the culture medium. The horizontal axis indicates time.

At three points in time samples of the culture supernatant were taken (indicated as MJ1, MJ2, and MJ3 in the figures).

### Example 2

Analogously to Example 1, LGG culturing was conducted on the basis of modified culture media. Herein Tween was absent, and an oil was added as follows:

| | |
|---|---|
| (a) Oleic acid | in 1 g/kg, 2 g/kg and 4 g/kg concentration. |
| (b) Linseed oil | in 1 g/kg, 2 g/kg and 4 g/kg concentration. |
| (c) Olive oil | in 1 g/kg, 2 g/kg and 4 g/kg concentration. |
| (d) Rape seed oil | in 1 g/kg, 2 g/kg and 4 g/kg concentration. |
| (e) Sunflower oil | in 1 g/kg, 2 g/kg and 4 g/kg concentration. |

In all of these tests (a)-(e), a successful LGG growth was observed, comparable to the growth in the Tween-containing medium. In addition to this, LGG surprisingly was also successfully cultured without the addition of Tween or any of the oils.

### Example 3

In this example, the supernatants obtained as in Example 1, were subjected to a screening for anti-allergic and anti-inflammatory activity using a RAW 264 cell (mouse macrophage cell line) *in vitro* model accepted in the art. The RAW cell cultures showed substantially increased production of the regulatory cytokine IL-10 during incubation with the MJ2 supernatant sample harvest of the LGG culture, as compared to the other supernatant sample harvests. See Fig. 2.

### Example 4

A comparison was made between the culture supernatant MJ2 obtained in Example 1 and viable LGG bacteria, in an ovalbumin (OVA) sensitation model. This *in vivo* model, well accepted in the art, is normally applied to adolescent or adult mice. In this Example, the conventional model was adapted so as to allow the study of allergy early in life.

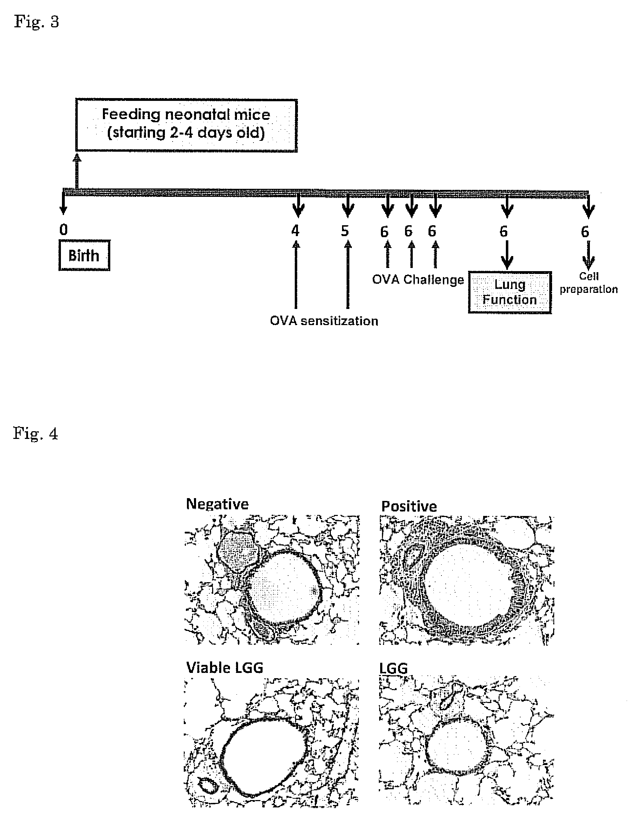

Thus, neonatal Balb/C mice received LGG or whole LGG culture supernatant every other day for six weeks through intragastric administration. Animals were sensitized to ovalbumin (OVA) twice at day 42 and 56 followed by later challenge and exposure to OVA-aerosol at days 61, 62, and 63. This time schedule is shown in Fig.3. Parameters of experimental bronchial asthma were assessed by lung function analyses, histology, and bronchoalveolar lavage (BAL). Systemic allergic reactivity was evaluated by antibody levels and cytokine responses. The latter was measured in the broncho alveolar lavage (BAL) as well as in re-stimulated draining lymph node cell cultures.

Exposure to both viable LGG as well as LGG supernatant was found to reduce airway inflammation and goblet cell hyperplasia. Histological staining of lung tissue sections (see Fig.4) showed increased inflammatory cell infiltrate and goblet cell hyperplasia in the OVA allergic animals whereas the LGG or LGG supernatant treated animals showed almost normal lung architecture and histology. In Fig. 4 "negative control" means: neither challenge, nor subject to LGG or LGG supernatant; "positive control" means: subjected to OVA challenge, not to LGG or LGG supernatant; "Viable LGG" means: subjected to challenge and to viable LGG; "LGG supernatant" means: subjected to challenge and to LGG supernatant treatment of the invention.

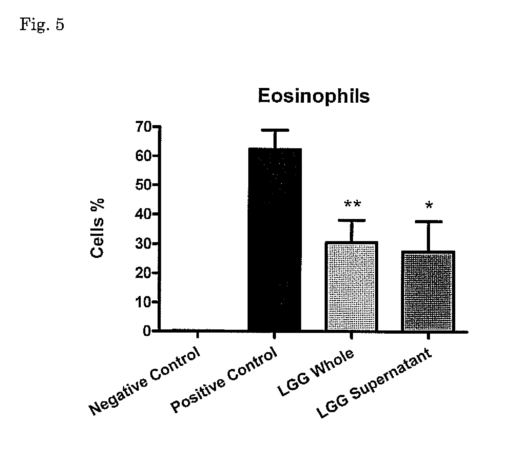

Further, the occurrence of infiltrating inflammatory cells (eosiniphils) was determined. Fig. 5 shows that the increased eosinophils infiltration in the lungs of OVA allergic animals was strongly reduced by treatment with viable LGG or LGG supernatant. In Fig.5, the vertical axis indicates the percentage of allergic cells' increase. On the horizontal axis the following samples are represented: "Negative control": no OVA challenge; no treatment; "Positive control": OVA challenge; no treatment; "LGG whole": OVA challenge followed by treatment with viable LGG; "LGG supernatant": OVA challenge followed by treatment with LGG supernatant of the invention.

### Example 5

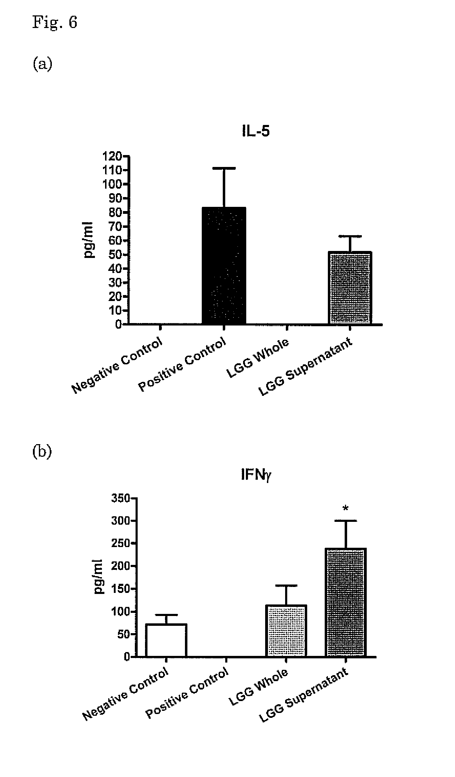

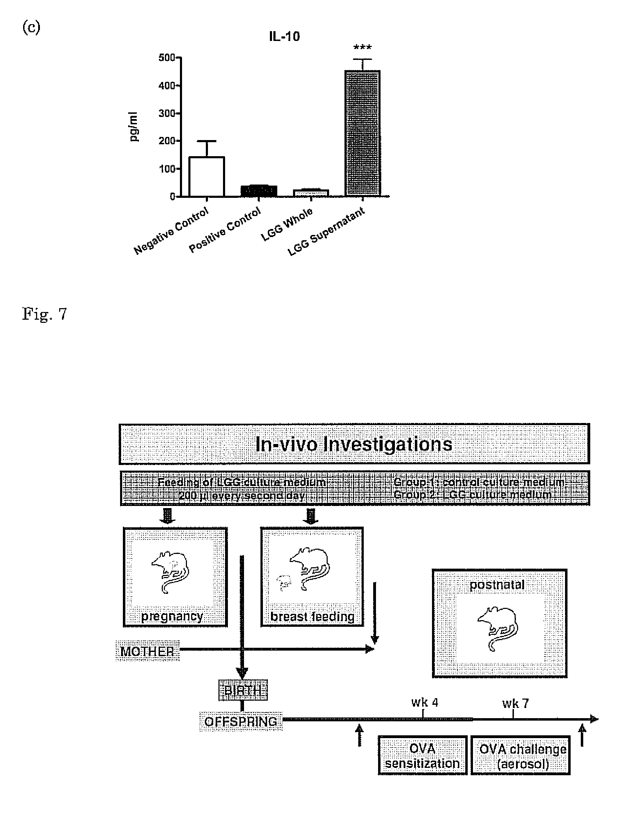

This example reflects an determination (conducted in a known manner) of the *in vitro* culture of cells isolated from lymph nodes. Typical Th2 cytokine profile in re-stimulated lymph node cell cultures from OVA allergic mice showed increased IL-5, and low IL-10 and IFN-γ responses. Treatment with either whole (viable) LGG or LGG supernatant of the invention revealed anti-allergic affects as revealed by decreased IL-5 response and strong stimulation of IFN-γ and IL-10 production in these cultures. This is depicted in Fig.6.

### Example 6

This example represents the perinatal administration of LGG culture supernatant of the invention in the ovalbumin (OVA) allergy model in Balb/C mice. Pregnant and lactating mothers received intragastric administration of LGG supernatant every other day. Their offspring were sensitized and challenged with OVA. A scheme for this test is illustrated in Fig. 7

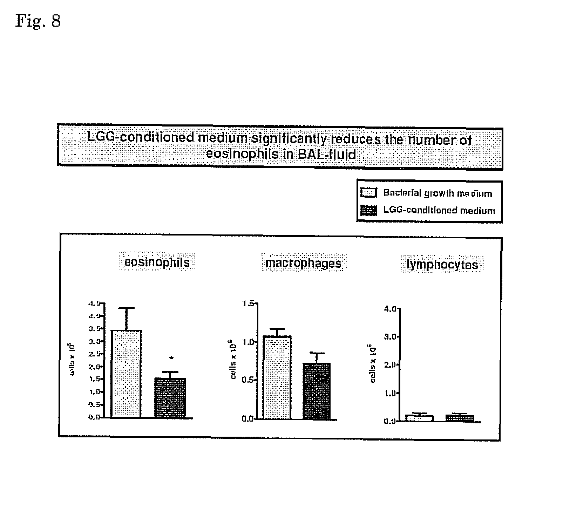

Parameters of experimental bronchial asthma were assessed by lung function analyses, histology, and bronchoalveolar lavage (BAL). Systemic allergic reactivity was evaluated by antibody levels and cytokine responses. The latter was measured both in BAL as well as in draining lymph node cultures. Fig. 8 indicates the results, in self-explanatory diagrams, revealing decreased infiltration of inflammatory cells (eosinophils, macrophages) in the LGG supernatant treated animals.

## Claims

1. A process for the preparation of a non-viable probiotic composition having anti-allergic and/or anti-inflammatory properties, the process comprising the steps of (a) subjecting LGG to cultivation in a suitable culture medium using a batch process; (b) harvesting the culture supernatant at a late exponential phase of the cultivation step, which phase is defined with reference to the second half of the time between the lag phase and the stationary phase of the LGG batch-cultivation process; (c) optionally removing low molecular weight constituents from the supernatant so as to retain molecular weight constituents above 6 kDa; (d) removing liquid contents from the culture supernatant so as to obtain the composition.

2. A process according to claim 1, wherein the late exponential phase is defined with reference to the latter quarter portion of the time between the lag phase and the stationary phase of the LGG batch-cultivation process, preferably 0.75-0.85 of the time elapsed in the exponential phase.

3. A process according to any one of the preceding claims, wherein the LGG batch cultivation is conducted in a culture medium devoid of Tween 80, the medium optionally containing an oily ingredient selected from the group consisting of oleic acid, linseed oil, olive oil, rape seed oil, sunflower oil, and mixtures thereof.

4. A process according to any one of the preceding claims, wherein the LGG batch cultivation is conducted at neutral pH, preferably pH 6, at physiological temperature, preferably 37°C.

5. A process according to any one of the preceding claims, wherein the supernatant is dried, preferably by spray-drying or freezedrying, with optionally.

6. A process according to claim 5, wherein a pharmaceutically acceptable carrier material, such as maltodextrin DE29, is added to the supernatant, followed by spray-drying.

7. A composition comprising a proteinaceous mixture obtainable from a culture supernatant in a late-exponential phase of an LGG batch-cultivation process, obtainable by a process in accordance with any one of the preceding claims.

8. A composition according to claim 7 in a dried form, preferably spray-dried or freeze-dried.

9. An infant or children's formula which is nutritionally complete with reference to the presence of lipids, carbohydrates, proteins, vitamins and minerals, further comprising a composition according to claim 8.

10. The use of a composition according to claim 7 or 8 in the manufacture of a medicament in the treatment or prevention of allergic and/or inflammatory diseases, including asthma, atopic eczema, food allergy, and allergic rhinitis/conjunctivitis.

11. A use according to claim 10, wherein the medicament is in the form of a dietetic product, preferably a nutritional product, or an additive for such a product.

12. The use of a composition according to claim 8 as an additive in a dietetic product, preferably a nutritional product.

13. A use according to claim 12, wherein the dietetic product further comprises one or more polyunsaturated fatty acids (PUFA's), preferably long-chain polyunsaturated fatty acids (LC-PUFA's) such as arachidonic acid (ARA) or docosahexaenoic acid (DHA).

14. A use according to claim 12 or 13, wherein the dietetic product further comprises one or more bio-active ingredients, such as proteins or polysaccharides, normally present in human breast milk.

15. A use according to any one of the claims 12 to 14, wherein the dietetic product further comprises one or more prebiotics, preferably selected from the group consisting of non-digestible oligosaccharides, non-digestible polysaccharides, and mixtures thereof.

16. A use according to any one of the claims 12 to 15, wherein the dietetic product is a prenatal, infant or children's formula or nutritional composition, a medical food, or a food for specific medical purposes.

17. A method of treatment or prevention of allergic and/or inflammatory diseases, comprising the administration of an effective amount of a composition according to claim 7 or 8 to a subject in need thereof.

18. The use of an oily substance selected from the group consisting of oleic acid, linseed oil, olive oil, rape seed oil, sunflower oil, and mixtures thereof, as a replacement of non-ionic emulsifiers such as polysorbates in a bacterial culture medium.

19. A use according to claim 18, wherein the bacterial culture medium serves to harvest a supernatant for application in infant formula or clinical nutrition.
